# EUROPEAN PATENT SPECIFICATION

(11) **EP 1 582 138 B1**
(45) Date of publication and mention of the grant of the patent: **10.09.2008**
(21) Application number: 05006143.1
(22) Date of filing: 21.03.2005
(51) Int. Cl.: A61B 1/018, A61B 19/00

(54) **Medical system with over-tube**
Medizinisches System mit Hüllrohr
Système medical avec tube permettant le guidage

(30) Priority: 02.04.2004 JP 2004110426
(43) Date of publication of application: 05.10.2005
(73) Proprietor: Olympus Corporation, Shibuya-ku, Tokyo (JP)
(72) Inventor: Okada, Yuta, Hachioji-shi Tokyo (JP); Sekine, Ryuta, Konagei-shi Tokyo (JP); Matsui, Raifu, Hino-shi Tokyo (JP)
(74) Representative: von Hellfeld, Axel

(56) References cited:
- US-A- 2 003 114
- US-A- 5 353 783
- US-A- 5 379 755
- US-A- 5 941 815
- US-A- 6 149 581
- US-B1- 6 361 488
- US-B1- 6 461 294
- US-B1- 6 508 759

## Description

### BACKGROUND OF THE INVENTION

### 1. Field of the Invention

The present invention relates to a medical system using an over-tube for guiding tools such as an endoscope, a flexible insertion guide tube, and the like into a body cavity, and a medical system using the over-tube.

### 2. Description of the Related Art

An endoscope treatment apparatus in which an instrument is guided into a body cavity through a flexible insertion guide tube and treatment is performed under the observation via the endoscope is known. In this endoscope treatment apparatus, the instrument insertion guide tube is guided into the body cavity through a so-called over-tube (see JP-A-2000-33071).

The instrument insertion guide tube of the endoscope treatment device in the related art described above is inserted into the over-tube and guided into the body cavity. Since the instrument insertion guide tube is retained by the over-tube, the position of the guide tube may be displaced during a surgical operation. When the position of the instrument insertion guide tube is displaced as described above, the positional relation between other instrument insertion guide tubes or the endoscope is also changed, and hence it is necessary to continue the surgical operation after having returned the position to the original position. In particular, when a relatively large load (force) is exerted to the instrument as in the case of pulling up the anatomy, the extent of movement of the instrument insertion guide tube is apt to be increased by a reaction thereof. Therefore, the operator may have trouble correcting the positional relation of the insertion guide tube.

In view of such a problem, it is an object of the present invention to solve such problem that the tools such as the instrument insertion guide tube guided through the over-tube are displaced during a surgical operation or inspection.

Arrangements relating to the use of endoscopes having a disposable outer sheath are described in US Patent No. 5,353,783 and US Patent No. 6,461,294. The US Patent No. 5,941,815 describes a splint or over-tube device for straightening the sigmoid colon of a patient during a colonoscopy. Similarly, US Patent No. 6,149,581 describes a tubular member that is releasably attachable to a colonoscope for insertion into the colon. The US Patent No. 6,361,488 describes a support apparatus for mounting an endoscope to assist a surgeon's manipulation of the endoscope during surgery. The US patent application published as US 2003/0114732 describes the use of an imaging instrument having balloon elements inserted through a sheath.

### BRIEF SUMMARY OF THE INVENTION

The present invention provides a medical system having the features as recited in claim 1. Preferred features of the invention are recited in the dependent claims.

The medical system of the invention includes an over-tube through which tools are inserted, and is provided with a restraining mechanism for restraining at least one of a relative movement between the tool inserted through the over-tube and the over-tube, or a relative movement of the over-tube and a body cavity. Finally, an unintended movement of the over-tube or the tools inserted through the over-tube may be reduced.

As one of the restraining mechanisms, there is an engagement mechanism for engaging the tool inserted through the over-tube at the distal end of the over-tube. According to this engagement mechanism, since the tool is engaged and fixed with respect to the over-tube, the movement of the tool with respect to the body cavity is restrained. Also, since the engagement mechanism engages the tool at the distal end of the over-tube, the length of the tool extended from the engaged position is short, and hence the movement can be restrained effectively. An operating mechanism for operating engagement and release thereof can be provided together with the engagement mechanism.

In this case, the operating mechanism can be provided on the over-tube on the proximal side so as to achieve good operability.

An engagement mechanism can include an elastic element and a pressing member for pressing the resilient strip toward the tool. For example, the resilient strip is deformed when a pressing force is exerted from the pressing member, and engages the tool. While when the pressing force is released, engagement of the tool is released. In this case, the pressing member can slide along the inner wall of the over-tube, and press the resilient strip during its sliding movement.

Alternatively, the engagement mechanism can be a balloon provided at the distal end of the over-tube. The balloon may be expanded and contracted by feeding and discharging fluid thereto/therefrom.

The operating mechanism and the engagement mechanism may be linked by a wire or a tube configured to allow fluid to flow therein.

When the over-tube includes a plurality of channels, the engagement mechanism can be provided to each channel, so as to be operated independently.

As another restraining mechanism, a supporting device for supporting the tool inserted through the over-tube at the outside of the body cavity may be provided. Since the tool is supported at the outside of the body cavity, the movement thereof can be restrained.

This support can be achieved by the use of at least two holders; a first holder for supporting the midsection of the tool, and a second holder for supporting the tool on the proximal side. The supporting positions of the respective holders can be configured to be variable. In particular, the position of the second holder can be changed with respect to the position of the first holder.

In this arrangement, during the surgical operation, change of the position or orientation of the tool can be performed by changing the position of the second holder while the position of the first holder is being fixed, whereby good efficiency is achieved.

A mechanism can also be provided for fixing the holder or releasing the fixation of the holder (for example, a brake) in supporting the holder.

As another restraining mechanism, it is conceivable to connect the endoscope to the distal end of the over-tube. By connecting the endoscope,unstability of the flexible over-tube is restrained, and consequently, the movement of the tool is restrained. This structure is good in workability since the distal end of the tool can be observed well by the endoscope.

### BRIEF DESCRIPTION OF THE SEVERAL VIEWS OF THE DRAWINGS

These and other features, aspects, and advantages of the apparatus and methods of the present invention will become better understood with regard to the following description, appended claims, and accompanying drawings where:
Fig. 1 is a perspective view of a state of usage of an endoscope treatment apparatus according to a first embodiment of the present invention;
Fig. 2 is a perspective view of an insertion guide tube of the first embodiment;
Fig. 3 is a cross section of the distal portion of the over-tube along the axis thereof according to the first embodiment;
Fig. 4 is a perspective view of the distal portion of the over-tube according to a second embodiment;
Fig. 5 is a perspective view of the portion of the over-tube in the vicinity of the proximal end according to the second embodiment.
Fig. 6 is an explanatory drawing of a device for supporting the portion of the over-tube in the vicinity of the proximal end according to an endoscope treatment apparatus of a third embodiment;
Fig. 7 is a perspective view of an over-tube according to the third embodiment;
Fig. 8 is a perspective view of the portion of the over-tube in the vicinity of the distal end according to an endoscope treatment apparatus of a fourth embodiment;
Fig. 9 is an explanatory drawing showing a state of usage of an over-tube supporting device in the endoscope treatment apparatus according to a fifth embodiment;
Fig. 10 is a perspective view of the supporting device according to the fifth embodiment;
Fig. 11 is a perspective view of a procedure of usage of the supporting device according to the fifth embodiment;
Fig. 12 is a perspective view of the distal portion of the over-tube in the endoscope treatment apparatus according to the fifth embodiment; and
Fig. 13 is an explanatory drawing showing a state of usage of the over-tube supporting device in the endoscope treatment apparatus according to a sixth embodiment.

### DETAILED DESCRIPTION OF THE INVENTION

Preferred embodiments of the invention will be described below with reference to the accompanying drawings.

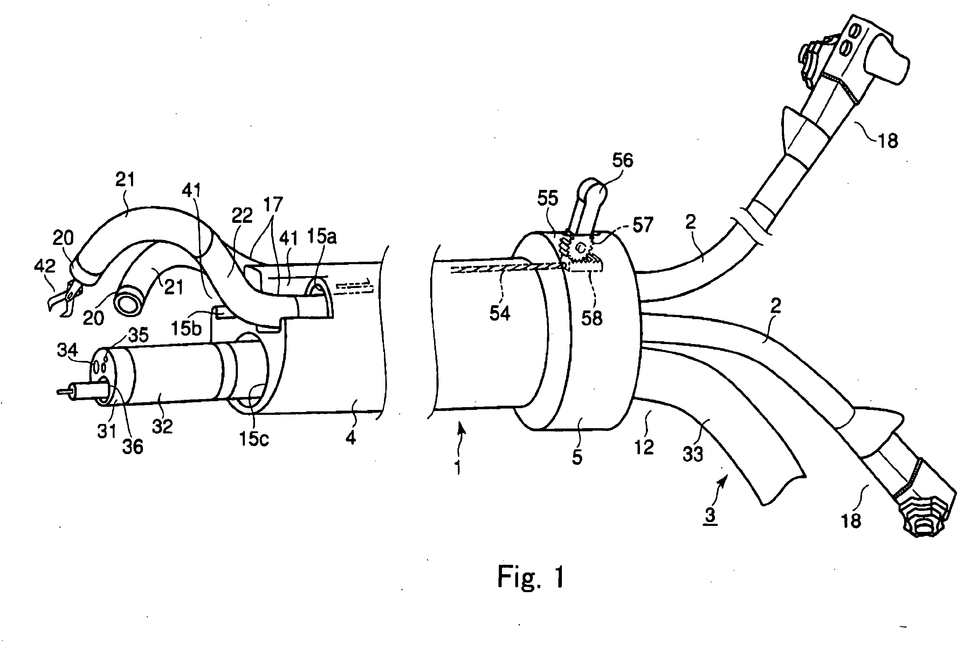

Referring to Fig. 1 to Fig. 3, an endoscope treatment apparatus according to a first embodiment of the invention will be described.

Fig. 1 is a perspective view showing a state of usage of the endoscope treatment apparatus according to the first embodiment. In Fig. 1, reference numeral 1 designates an over-tube. The over-tube 1 is formed of a flexible tubular member having a plurality of channels (lumens) 15a, 15b, 15c for allowing an instrument insertion guide tube 2, an endoscope 3, or various tools such as an instrument to be inserted for guiding these tools into a body cavity. The over-tube 1 includes an insertion portion 4 and a proximal portion 5. In this case, separate tools are to be inserted into the channels 15a, 15b, 15c. The instrument insertion guide tubes 2 are inserted into the channels 15a, 15b and the endoscope 3 is inserted into the channel 15c.

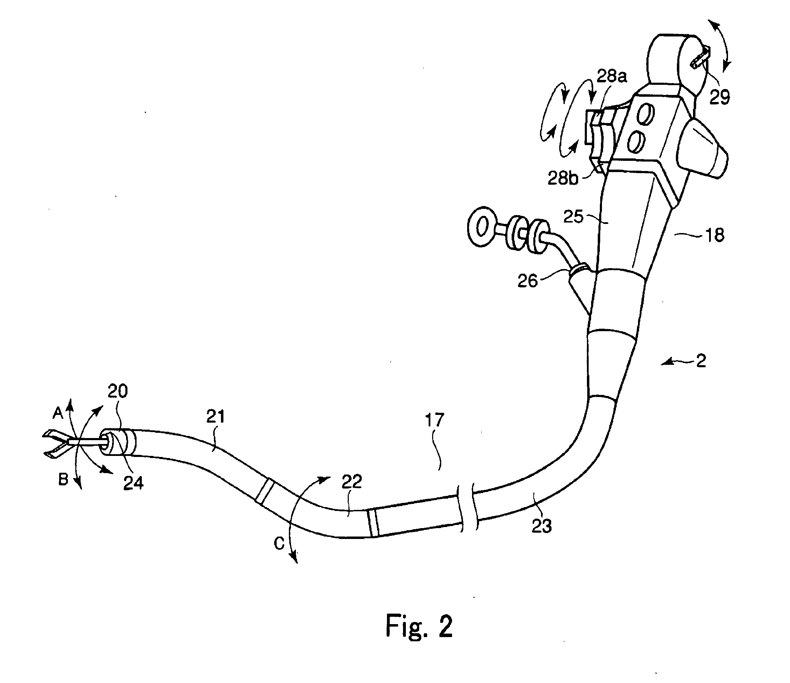

Fig. 2 is a perspective view of the instrument insertion guide tube 2. As shown in Fig. 2, each of the instrument insertion guide tubes 2 includes a flexible insertion portion 17, and an operating member 18 provided on the proximal portion of the insertion portion 17. The insertion portion 17 includes a distal portion 20 located at the extremity thereof, a first bending portion 21 located on the proximal side of the distal portion 20, a second bending portion 22 located on the proximal side of the first bending portion 21, and a flexible portion 23 provided behind the second bending portion 22. The insertion portion 17 of the instrument insertion guide tube 2 is formed with an instrument guiding channel 24 therein along the entire length thereof. The instrument guiding channel 24 is opened toward the outside at the extremity of the distal portion 20 thereof. The first bending portion 21 is adapted to bend in any of upward, downward, leftward and rightward directions as shown by arrows A and B in Fig. 2. The second bending portion 22 is adapted to bend only in the upward and downward directions as shown by arrow C in Fig. 2. These bending portions 21, 22 are adapted to be bent by a plurality of bending operating wires (not shown) as is known in the art.

As shown in Fig. 2, the operating member 18 on the proximal side includes a gripping portion 25, an insertion port 26 in communication with the instrument guiding channel 24. The operating member also includes, as a plurality of operating members for bending the bending portions 21 and 22, angle knobs 28a, 28b for operating the first bending portion 21, and an angle handle 29 for operating the second bending portion 22.

As shown in Fig. 1, the insertion portion 12 of the endoscope 3 includes a distal portion 31 located at the extremity thereof, a bending portion 32 located on the proximal side of the distal portion 31, and a flexible portion 33 located on the proximal side of the bending portion 32. The distal portion 31 is provided with an observation window 34, an illumination window 35, a channel port 36, and so on at the distal end surface thereof. The bending portion 32 is bent by the operating member (not shown) provided at a proximal end of the insertion portion 12.

As shown in Fig. 1, in the over-tube 1, the distal openings 41 of the channels 15a, 15b for allowing the instrument insertion guide tubes 2 to be inserted for guiding the same are not only opening toward the front, but also opening continuously and widely toward the sides. Therefore, the over-tube 1 is adapted so as not to impair the bending motion of the bending portions 21, 22 of the instrument insertion guide tubes 2.

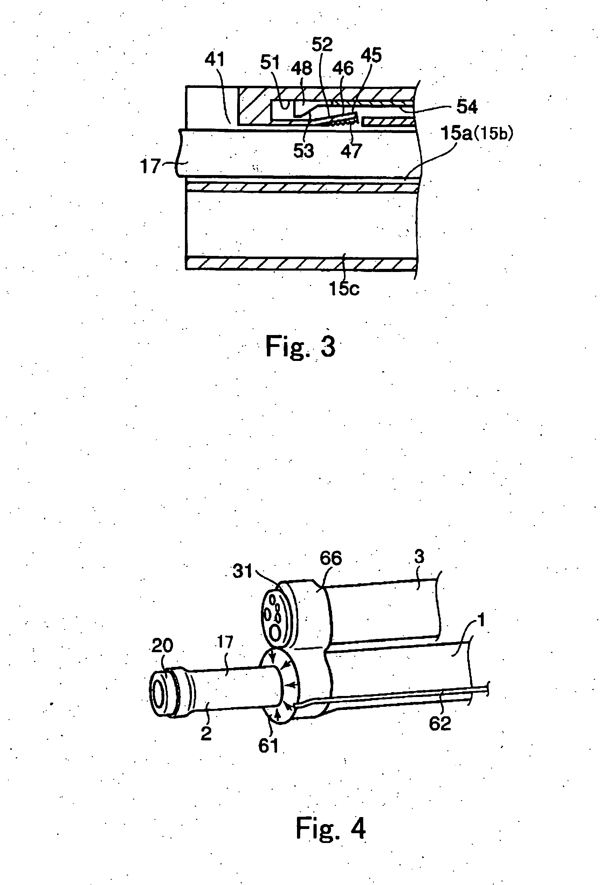

Fig. 3 is a cross section of the distal portion of the over-tube 1 along the axis thereof. As shown in Fig. 3, the distal opening 41 of the channel 15a (15b) is provided therein with an engaging member for pressing against the insertion portion 17 of the instrument insertion guide tube 2 inserted into the channel 15a (15b), and releasably and fixedly engaging the insertion portion 17 of the instrument insertion guide tube 2 to the over-tube 1. In other words, there is provided a stopper 45 for fixing the insertion portion 17 of the instrument insertion guide tube 2 in position. The stopper 45 includes a resilient strip 46 formed along the inner wall of the channel 15a (15b), and a frictional member 47 attached to the distal end of the resilient strip 46. The resilient strip 46 is normally retracted at a position not projecting into the passage of the channel 15a (15b) as shown in Fig. 3. However, when the resilient strip 46 is pushed out into the channel 15a (15b) by a pressing member 48, described later, it is brought into abutment and engagement with the insertion portion 17 of the instrument insertion guide tube 2 inserted in the channel 15a (15b), and hence retaining the insertion portion 17 of the instrument insertion guide tube 2 in position with respect to the over-tube 1. The engagement mechanism as described above (position retaining mechanism) may be provided also in the channel 15c for engaging the insertion portion 12 of the endoscope 3.

As shown in Fig. 3, the pressing member 48 is installed so as to be capable of sliding in the fore-and-aft direction of the over-tube 1 along a guiding surface 51 formed along the inner wall of the channel 15a (15b) opposing the backside of the resilient strip 46. The pressing member 48 is formed with a projection 53 which comes into abutment with an inclined surface 52 formed on the back surface of the resilient strip 46. Then, an operating wire 54 is connected to the pressing member 48, so that the pressing member 48 can be slid by the operating wire 54. The operating wire 54 is guided to the proximal portion 5 provided on the proximal portion through the interior of the over-tube 1.

As shown in Fig. 1, a wire operating mechanism 55 as an operating mechanism for moving the operating wire 54 back and forth is built in the proximal portion 5 of the over-tube 1. The wire operating mechanism 55 includes a pinion gear 57 to be rotated by an operating lever 56 and a rack 58 that meshingly engages and is slid by the pinion gear 57, and the operating wire 54 is connected to the rack 58. By operating the wire operating mechanism 55 by rotating the operating lever 56, the operating wire 54 can be moved back and forth. In this case, the wire operating mechanisms 55 of the stoppers 45 of the respective channels 15a, 15b are operated by the single operating lever 56. However, it is also possible to configure in such a manner that the operating levers 56 as the operating means are provided independently to the respective stoppers 45 of the respective channels 15a, 15b, and the insertion portions of the tools inserted through the channels 15a, 15b are releasably engaged independently therewith.

Subsequently, the case of using the endoscope treatment apparatus described above will be described. Firstly, the operator inserts the insertion portion 12 of the endoscope 3 through the over-tube 1, causes the distal portion of the insertion portion 12 to project from the distal end of the over-tube 1 in advance, and inserts the portion of the insertion portion 12 of the endoscope 3 having an observing function into the body cavity such as the inside of stomach. Subsequently, the operator inserts the over-tube 1 into the body cavity while laying the over-tube along the insertion portion 12 of the endoscope 3. Once the over-tube 1 is inserted into the body cavity, the tools such as the endoscope 3 can be inserted and removed therein/therefrom as needed.

Then, the interior of the body cavity can be observed by the endoscope 3 inserted into the body cavity through the over-tube 1 for diagnosis or surgical operation. When performing diagnosis or surgical operation by the use of an instrument 42, the operator inserts the instrument insertion guide tube 2 through the channels 15a, 15b of the over-tube 1 as shown in Fig. 1, causes the movable portion on the distal side including the bending portions 21, 22 of the instrument insertion guide tubes 2 to project from the distal opening 41 of the over-tube 1, and inserts the instrument 42 into the interior of the body cavity through the instrument insertion guide tubes 2.

Subsequently, the operator operates the operating lever 56 on the proximal portion 5 of the over-tube 1, and fixes the instrument insertion guide tubes 2 to the over-tube 1 by the stoppers 45. Accordingly, the position of the instrument insertion guide tubes 2 with respect to the over-tube 1 is determined, and hence the positions of the instrument insertion guide tubes 2 do not change. In this state, the operator bends the bending portions 21, 22 of the instrument insertion guide tubes 2, and determines the orientation or the position of the instrument 42 before conducting the surgical operation.

In this manner, since the positions of the instrument insertion guide tubes 2 with respect to the over-tube 1 do not change, it is no longer necessary to adjust the positions of the instrument insertion guide tubes 2 during the surgical operation.

When the plurality of the instruments 42 are used by the use of the plurality of the instrument insertion guide tubes 2 as in this embodiment, the position of one of the instrument insertion guide tubes 2 which guides one of the instruments 42 is apt to change while the other instrument 42 is being operated. However, since the instrument insertion guide tube 2 can be fixed to the over-tube 1, this inconvenience can be avoided. When pulling up the tissue with the instrument 42, even when a relatively large load is exerted to the instruments 42 and the instrument insertion guide tubes 2 which operate the instruments 42, the instrument insertion guide tube 2 is prevented from being displaced by the reaction therefrom.

As described above, according to the first embodiment, movement of the instrument insertion guide tubes 2 guided through the over-tube 1 can be restrained by using the engagement mechanism that is capable of engagement and release operations.

Although the case in which the instrument insertion guide tubes 2 are fixed to the over-tube 1 has been described in the first embodiment, it is also possible to use the similar fixing engagement mechanism and fix the insertion portion of the endoscope 3 to the over-tube 1.

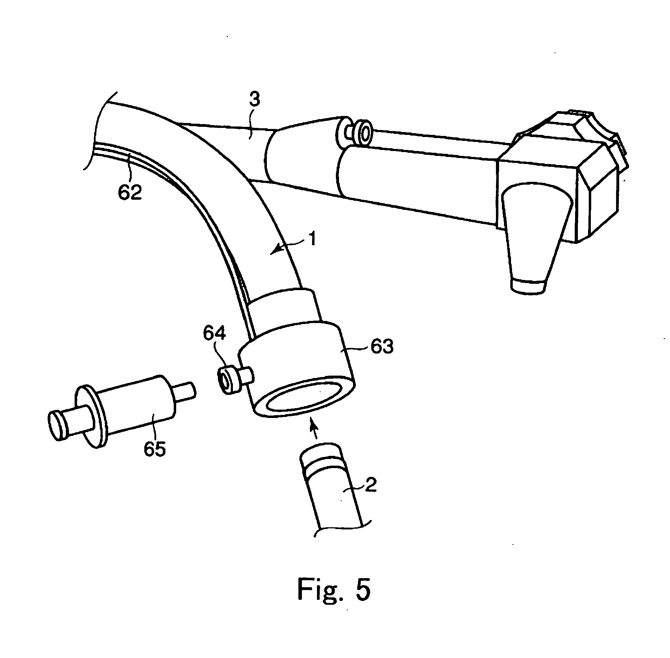

Subsequently, referring now to Fig. 4 and Fig. 5, the endoscope treatment apparatus according to a second embodiment will be described. The same parts as those in the above-described embodiment will be represented by the same reference numerals.

Fig. 4 is a perspective view of the distal portion of the over-tube according to a second embodiment. The second embodiment employs a fluid-operating type engagement mechanism in which a balloon 61 is attached along at least a portion of the circumference of a peripheral edge of the distal opening of the insertion guide tube channel formed in the over-tube 1. The balloon 61 is expanded for pressing the insertion portion 17 of the instrument insertion guide tube 2, so that the instrument insertion guide tube 2 is fixedly engaged with the over-tube 1. The balloon 61 is connected to a tube 62 disposed on the outer periphery or inside the over-tube 1, so that the fluid can be supplied or discharged to/from the balloon 61 through the tube 62 (fluid conduit).

Fig. 5 is a perspective view of the portion of the over-tube in the vicinity of the proximal end thereof according to the second embodiment. As shown in Fig. 5, the tube 62 is guided to the proximal portion 63 of the over-tube 1 located outside the body along the outer periphery of the over-tube 1, and is connected to a connecting portion 64 provided on the proximal side 63 thereof. Then, a syringe 65 is connected to the connecting portion 64, and the fluid is supplied and discharged to/from the balloon 61 using the syringe 65.

As shown in Fig. 4, in the second embodiment, a connecting member 66 mounted to the distal end portion of the over-tube 1 detachably retains the distal portion 31 of the insertion portion of the endoscope 3. When the connecting member 66 is used, the operator can align the insertion portion of the endoscope 3 along the over-tube 1, engage the distal portion 31, which is the portion of the endoscope 3 located on the distal side of the bending portion, with the distal end of the over-tube 1 with the connecting member 66, and guide the both of them together into the body cavity. Also, in this state, the operator can observe the state of the instrument insertion guide tube 2 projecting from the distal end of the over-tube 1 or of the instrument 42 by the endoscope 3.

As shown in Fig. 4, the operator inserts the insertion portion 17 of the instrument insertion guide tube 2 into the over-tube 1 and causes the distal portion 20 to project by a suitable amount. Then, after having determined the position of the instrument insertion guide tube 2 in the fore and aft direction, the fluid is supplied to the balloon 61 to expand the balloon 61, so that the insertion portion 17 of the instrument insertion guide tube 2 can be fixed with respect to the over-tube 1 at this position. By keeping the balloon 61 in the contracted state, the distal opening of the over-tube 1 is released, and hence the back and forth movement of the instrument insertion guide tube 2 is allowed. In the case of the second embodiment as well, as in the case of the first embodiment, the back and forth movement and the fixation of the instrument insertion guide tube 2 with respect to the over-tube 1 are possible. Therefore, with the second embodiment as well, the same effects as in the first embodiment described above are obtained.

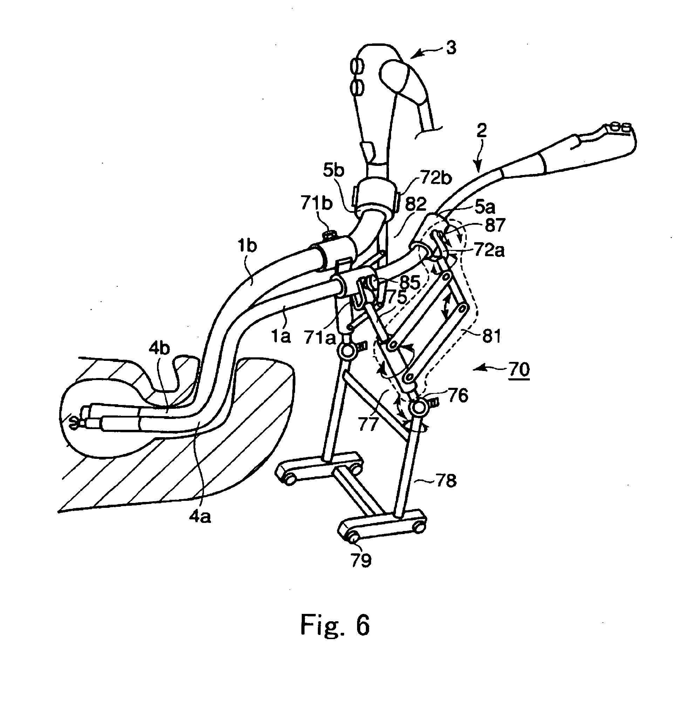

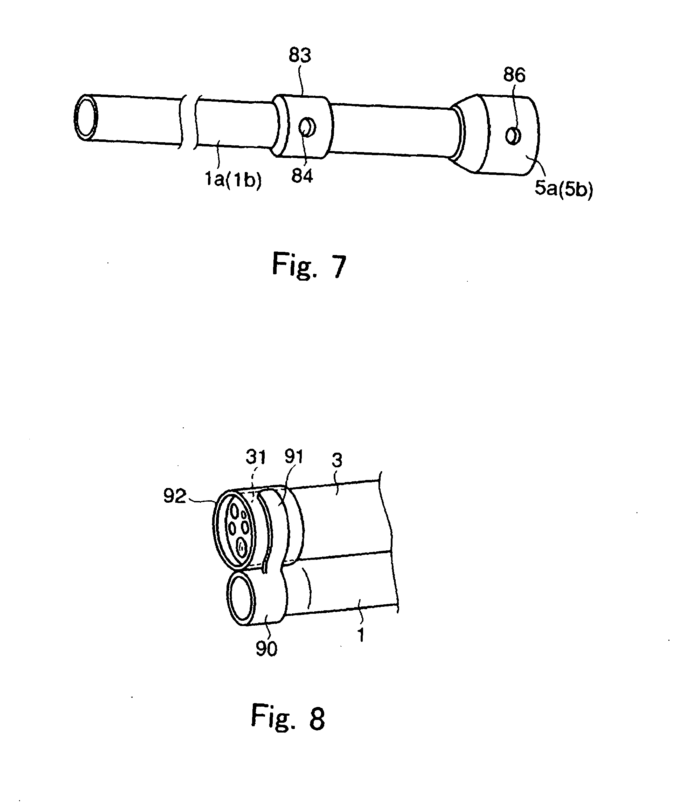

Referring now to Fig. 6 and Fig. 7, the endoscope treatment apparatus according to a third embodiment of the present invention will be described. The same parts as in the first and second embodiments are represented by the same reference numerals.

Fig. 6 is an explanatory drawing of a device for supporting the portion of the over-tube in the vicinity of the proximal end in the endoscope treatment device according to the third embodiment. In the third embodiment, a supporting device 70 for fixing the proximal portion of the over-tube as described above in position at a plurality of points is employed. In other words, in the supporting device 70, the first over-tube 1a for guiding the instrument insertion guide tube 2 includes a first fixed holder 71a for gripping the midsection of the proximal portion of the insertion portion 4a and a first movable holder 72a for gripping the proximal portion 5a thereof. In the supporting device 70, the second over-tube 1b for guiding the insertion portion of the endoscope 3 includes a second fixed holder 71b for gripping the midsection on the proximal side of the insertion portion 4b and a second movable holder 72b for gripping the proximal portion 5b.

The first fixed holder 71a and the second fixed holder 71b are both supported by a base member 78 so that the position to be supported thereof can be selected arbitrarily by a supporting arm 77 having an expansible link 75 or a spherical surface bearing 76 which allows free rotation or fixation. The supporting arm 77 is adapted to be capable of changing the position to support the fixed holders 71a, 71b, which constitutes a first supporting position adjusting mechanism. The base member 78 is provided with casters 79, which allows the device to be moved freely on the floor.

On the other hand, the first movable holder 72a is movably supported by the first fixed holder 71a via a first link mechanism 81. The second movable holder 72b is movably supported by the second fixed holder 71b via a second link mechanism 82. Therefore, the first movable holder 72a and the second movable holder 72b can move while retaining the proximal portions 5a, 5b of the over-tubes 1a, 1b about the fixed holders 71a, 71b as fulcrums or reference points. The first link mechanism 81 and the second link mechanism 82 are adapted to be capable of changing the supporting positions of the movable holders 72a, 72b, which constitute a second supporting position adjusting mechanism.

The movable portions of the first link mechanisms 81, 82 of the supporting device 70 are each integrally provided with a frictional force generating mechanism (not shown) for applying a resistance to the movement thereof. In other words, even when the operator releases his/her hand in a state of supporting the instrument insertion guide tube 2 or the endoscope 3, the movable holders 72a, 72b are fixed at their positions. The movable portion may also be provided integrally with, for example, an electromagnetic brake, which can selectively switch between the fixed state in which a frictional force is applied, and the fixation released state in which the movable portion can move freely. The operation of the electromagnetic brake may be operated by operating means such as a switch provided, for example, on the supporting device 70 or the periphery thereof.

Alternatively, it is also possible to configure in such a manner that when the movable portions of the first link mechanisms 81, 82, being in a fixed state, is applied with a slightly larger force, the first link mechanisms 81, 82 is brought into a movable fixed state, so that the movable holders 72a, 72b can be moved in a state of supporting the proximal portions 5a, 5b of the instrument insertion guide tubes 2 and the endoscope 3.

Fig. 7 is a perspective view of the over-tube according to the third embodiment. As shown in Fig. 7, a cylindrical retaining member 83 is mounted to the midsection of the insertion portion 4a (4b) of the over-tube 1a (1b) and a setscrew 85 of the fixed holder 71a (71b) is fitted to and engaged with an engaging hole 84 formed on the retaining member 83. Further, an engaging hole 86 is formed at the proximal side 5a (5b) of the over-tube 1a (1b). A screw 87 of the movable holders 72a (72b) is screwed and fixed to the engaging hole 86.

In the third embodiment, since the proximal portions of the over-tubes 1a, 1b can be supported by the supporting device 70, it is easy to replace the over-tubes 1a, 1b. Since the midsection on the proximal side of the flexible insertion portions 4a, 4b of the over-tubes 1a, 1b are gripped by the fixed holders 71a, 71b, and the positions with respect to the patient are determined, thereby being supported fixedly in position, the positions of the distal ends of the tools such as the instrument insertion guide tube 2 or the endoscope 3 can be stabilized. Also, since the proximal portions 5a, 5b of the over-tubes 1a, 1b are gripped by the separate movable holders 72a, 72b, they can be moved freely according to the processing state of the surgical operation and retained at an optimal position for operation. Therefore, the operational performance of the instrument insertion guide tube 2 or the endoscope 3, these are inserted in to the over-tubes 1a, 1b, can be enhanced. Also, since the supporting positions of the fixed holder 71, 71b and the movable holders 72a, 72b are apart from each other and flexible insertion portions exist between the fixed holders 71a, 71b and the movable holders 72a, 72b, stability of the distal portions of the insertion portions 4a, 4b near the patient increases. Also, owing to the movable holders 72a, 72b, when inserting the instrument insertion guide tube 2 or the endoscope 3 through the over-tubes 1a, 1b, the operation at the proximal side for moving the proximal portions 5a, 5b can be performed easily. Also, owing to the fixed holders 71a, 71b, the influence of the operation of the proximal portions 5a, 5b is prevented from being transmitted to the distal sides. In this arrangement as well, the distal portions of the flexible insertion portions 4a, 4b of the over-tubes 1a, 1b can be stabilized.

Furthermore, since the proximal portions 5a, 5b of the over-tubes 1a, 1b are retained by the movable holders 72a, 72b positioned on a far side from the patient with respect to the fixed holders 71a, 71b, even when the operator removes his/her hand from the proximal portions 5a, 5b of the instrument insertion guide tube 2 or the endoscope 3, the posture can be maintained as is.

The supporting position of the instrument insertion guide tube 2 or the endoscope 3 by the fixed holders 71a, 71b are not changed during the surgical operation. It is for stabilizing the position of the distal end of the operational tool. However, the positions of the fixed holders 71a, 71b can be changed by operating the supporting arm 77.

On the other hand, the proximal portions 5a, 5b of the instrument insertion guide tube 2 or the endoscope 3 can be maintained in place by the movable holders 72a, 72b even when the operator removes his/her hand. However, when the proximal portions 5a, 5b are allowed to move by releasing the fixed state and selecting the free state, or by exerting an external force in the fixed state, if a rather large force is exerted, the movable holders 72a, 72b can be moved so as to follow the movement of the instrument insertion guide tube 2 or the endoscope 3.

In the over-tube of the third embodiment, the tube fixing mechanism such as the stopper mechanism in the above-described first or second embodiment can be integrated.

Referring now to Fig. 8, the endoscope treatment apparatus according to a fourth embodiment will be described. The same parts as in the embodiments described above will be represented by the same reference numerals.

Fig. 8 is a perspective view of the portion of the over-tube in the vicinity of the distal end thereof in the endoscope treatment apparatus according to the fourth embodiment of the invention. In the fourth embodiment, the over-tube 1 is provided with a connecting member 90 at the distal end thereof, and the connecting member 90 is formed with a gripping strip 91 for retaining the distal portion 31 of the insertion portion of the endoscope 3. A hood 92 mounted to the distal portion 31 of the endoscope 3 is gripped by the gripping strip 91, and the distal ends of the over-tube 1 and the endoscope 3 are connected. In this arrangement, the state of usage of the tools such as the instrument insertion guide tube 2 or the instrument 42 projecting from the distal end of the over-tube 1 can easily be observed by the endoscope 3. The tube fixing means such as the stopper mechanism in the first or second embodiment described above can be integrated and applied in the over-tube in the fourth embodiment.

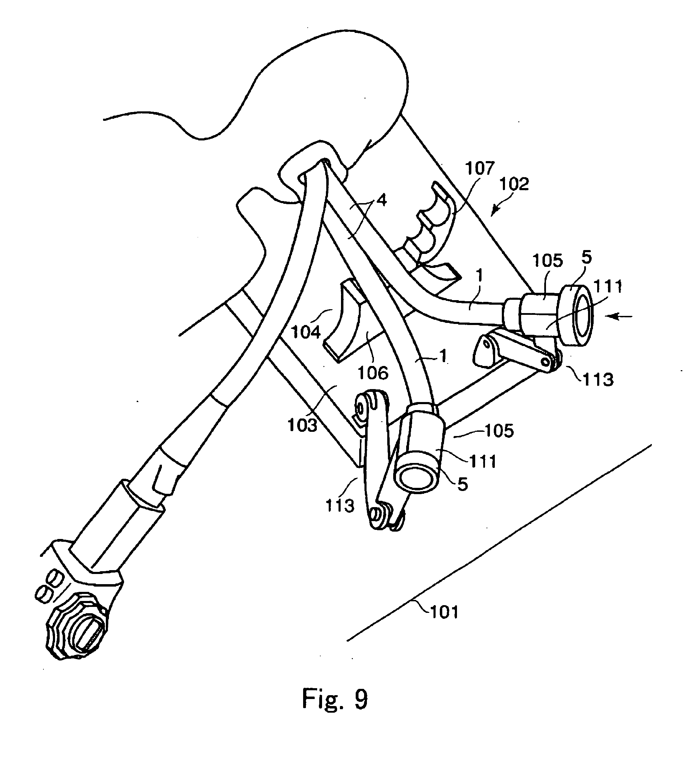

Fig. 9 to Fig. 12 show the endoscope treatment apparatus according to a fifth embodiment of the invention. The same parts as in the embodiments described above are represented by the same reference numerals.

Fig. 9 is an explanatory drawing showing a state of usage of the over-tube in the endoscope treatment apparatus according to the fifth embodiment. The fifth embodiment is an example of an over-tube supporting device 102 which can be placed on a patient's bed 101. As shown in Fig. 9, the supporting device 102 includes a base member 103, and the base member 103 includes one fixed-type first supporting member 104 for placing the midsections of the insertion portions 4 of the two over-tubes 1, and a plurality of movable type second supporting members 105 for supporting the proximal portions 5 of the over-tube 1 independently. The first supporting member 104 is disposed near the patient, so that the plurality of the over-tubes 1 can be retained together. The first supporting member 104 includes a base 106 for receiving the over-tubes 1 from below, and a lid-shaped holding member 107 which can be turned upward for opening and closing the first supporting member 104. The first supporting member 104 and the holding member 107 have concavities conforming the shapes of the over-tubes 1. When the over-tubes 1 are placed within the concavities of the supporting member 104, the holding member 107 is turned down to hold and retain the over-tubes 1. The both members are locked together with a mechanism well known in the art to firmly secure the over-tubes 1.
The second supporting members 105 are disposed apart from each other, and are also disposed apart from the first supporting member 104.

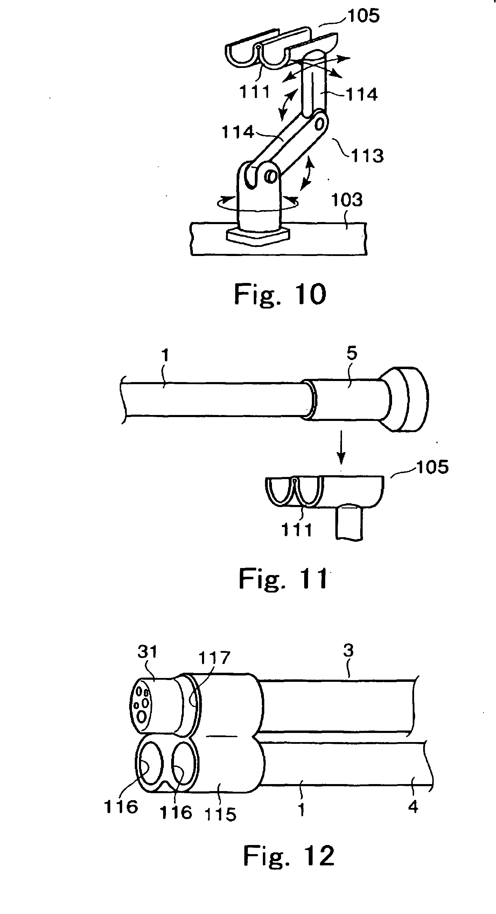

Fig. 10 is a perspective view of the supporting device according to the fifth embodiment. As shown in Fig. 10, the second supporting member 105 includes a gripping member 111 for gripping the proximal portion 5 of the over-tube 1, which can be opened and closed. The semi-circular portions of supporting member 105 are locked together in the closed position with a mechanism well known in the art. Fig. 11 shows a state in which the gripping member 111 is opened for placing the proximal portion of the over-tube 1 thereon.

The gripping member 111 is retained at the distal end of a supporting arm 113 mounted to the base member 103. The supporting arms 113 can change the position of the gripping member 111 freely by varying the direction of rotation of respective arms 114 and the gripping member 111 by rotatably connecting a plurality of the arms 114 and the gripping member 111 in sequence. The respective connecting portions of the supporting arms 113 are provided, for example, with electromagnetic brakes (not shown) by which the operator can apply and release a predetermined frictional force as needed, so that the relative movement of the supporting arm 113 is allowed and restrained by selectively operating the brakes when necessary.

Fig. 12 is a perspective view of the portion of the over-tube in the vicinity of the distal end in the endoscope treatment apparatus according to the fifth embodiment. Fig. 12 shows an example of a connecting member 115 for bundling the distal ends of the two over-tubes 1 and the distal portion 31 of the insertion portion of the endoscope 3. The connecting member 115 includes two insertion holes 116 for fitting the distal portions of the two over-tubes 1 independently, and an insertion hole 117 for fitting the distal portion 31 of the endoscope 3.

In this manner, in the fifth embodiment, since the proximal portion of the over-tube 1 can be supported by the supporting device 102, it is easy to handle the over-tube 1. Also, the midsections of the flexible insertion portions 4 of the over-tube 1 are gripped by the first supporting member 104 and the proximal portions 5 are gripped by the separate connecting members 115. Therefore, the portion of the flexible insertion portion 4 close to the patient can be fixedly supported. The first supporting member 104 and the second supporting member 105 are disposed at a distance, the instrument insertion guide tubes 2 or the flexible insertion portion of the endoscope 3 exist therebetween. Therefore, the influence of operation such as insertion of the instrument insertion guide tube 2 through the over-tube 1 or movement of the proximal portion 5 of the endoscope 3 can be blocked, whereby the distal side of the flexible insertion portion 4 of the over-tube 1 can be stabilized. Furthermore, since the proximal portion 5 of the over-tube 1 is gripped by the freely-movable second supporting member 105, operation to move the instrument insertion guide tube 2 inserted through the over-tube 1 or the proximal portion 5 of the endoscope 3 can be moved freely and hence the operability of the instrument insertion guide tube 2 or the endoscope 3 can be ensured.

The tube fixing mechanism such as the stopper mechanism in the first and second embodiment described above can be integrated and applied to the over-tube 1 of the fifth embodiment.

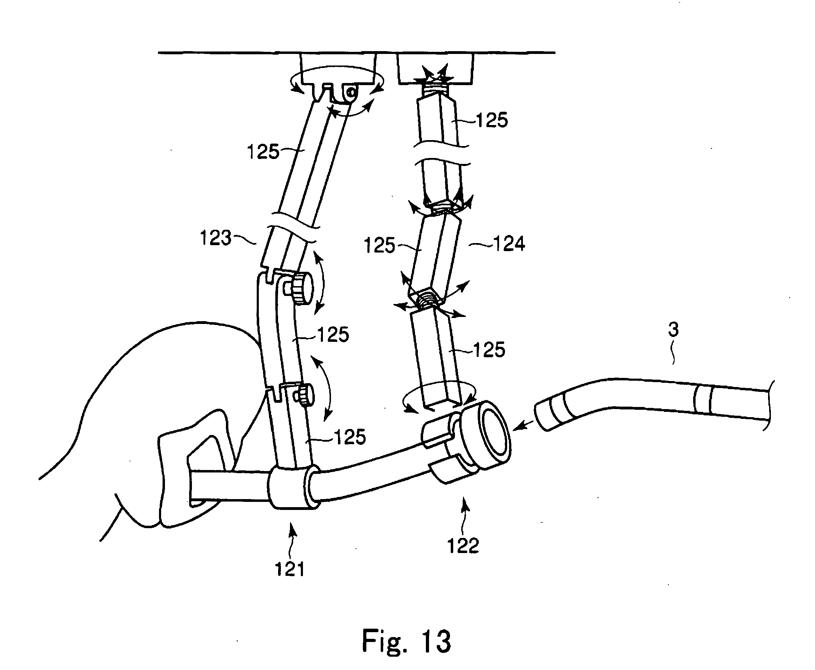

Fig. 13 shows a supporting device according to a sixth embodiment of the invention. The same parts as in the embodiments described above are represented by the identical reference numerals.

In this embodiment, a first supporting member 121 for retaining the midsection of the insertion portion 4 of the over-tube 1 and a second supporting member 122 for supporting the proximal portion 5 of the over-tube 1 are provided. The first supporting member 121 is disposed in the vicinity of the patient, and the second supporting member 122 is disposed apart from the first supporting member 121.

Both of the first supporting member 121 and the second supporting member 122 are retained respectively by the distal ends of the separate supporting arms 123, 124 suspended overhead from a fixed structure, such as from the ceiling. Both of the supporting arms 123, 124 are adapted to change the positions of the supporting members 121, 122 freely by differentiating the direction of rotation of the respective arms 125 as needed by connecting a plurality of arms 125 in sequence as shown in Fig. 13. The connecting portions of the supporting arms 123, 124 are provided with brakes (not shown) by which the operator can apply and release a predetermined frictional force as needed, so that the relative movement of the supporting arms 123, 124 is allowed or restrained by operating these brakes when necessary.

The sixth embodiment has the same effects as those described above although suspending the supporting members from a fixed overhead structure, such as the ceiling is different. Since the supporting members 121, 122 are suspended from overhead, such as from the ceiling, these members can hardly be a hindrance. The tube fixing means such as the stopper mechanism in the embodiments described above can be integrated and applied to the over-tube according to the sixth embodiment.

The invention is not limited to those described above, and may be applied to other embodiments.

While there has been shown and described what is considered to be preferred embodiments of the invention, it will, of course, be understood that various modifications and changes in form or detail could readily be made. It is therefore intended that the invention be not limited to the exact forms described and illustrated, but should be constructed to cover all modifications that may fall within the scope of the appended claims.

## Claims

1. A medical system comprising an over-tube (1) having at least one channel (15a, 15b, 15c) extending from a proximal portion (5) to a distal portion (4) and having a distal opening (41) through which a tool (2, 3) is able to be inserted and guided into a body cavity,
**characterized in that** the over-tube (1) comprises a restraining mechanism (45, 54; 61, 62) for restraining a relative movement between the tool (2, 3) inserted through the over-tube (1) and the over-tube (1), whereby a desired position of the tool (2, 3) projecting from the distal opening (41) of the over-tube (1) is fixable relative to the over-tube (1) by an operator.

2. A medical system according to Claim 1, wherein the restraining mechanism comprises:
an engagement mechanism (45; 61) provided at a distal portion of the over-tube for releasably engaging the tool inserted through the over-tube with the over-tube; and
an operating mechanism (54-58; 62-65) for operating engagement and release of the engagement mechanism.

3. A medical system according to Claim 2, wherein the operating mechanism (54-58; 62-65) is provided on a proximal side of the over-tube.

4. A medical system according to Claim 2, wherein the engagement mechanism (45, 54) comprises:
a resilient strip (46) disposed at the distal portion of the over-tube; and
a pressing member (48) for pressing the resilient strip (46) against the tool (2, 3) inserted through the over-tube (1).

5. A medical system according to Claim 4, wherein the resilient strip (46) engages the tool (2, 3) by being deformed by depression by the pressing member (48), and releases the engagement by being restored by releasing of the depression.

6. A medical system according to Claim 5, wherein the pressing member (48) presses the resilient strip (46) and releases depression from the resilient strip by sliding movement along the inner wall of the channel (15a, 15b, 15c) of the over-tube (1), and wherein the operating mechanism (54-58) operates the sliding movement of the pressing member (48).

7. A medical system according to Claim 2, wherein the engagement mechanism includes a balloon (61) disposed at the distal end of the over-tube.

8. A medical system according to Claim 7, wherein the operating mechanism (62-65) feeds and discharges fluid to and from the balloon (61).

9. A medical system according to Claim 2, wherein the operation of the operating mechanism (54-58) is transmitted to the engagement mechanism via a wire (54).

10. A medical system according to Claim 2, wherein the operation of the operating mechanism (62-65) is transmitted to the engagement mechanism via a tube (62) through which a fluid is fedable and dischargeable.

11. A medical system according to Claim 2, wherein the over-tube (1) includes a plurality of channels (15a, 15b, 15c) through which a plurality of tools (2, 3) can be inserted, and wherein the engagement mechanism (45; 61) is provided in at least one of the channels (15a, 15b, 15c).

12. A medical system according to Claim 11, wherein the engagement mechanism (45; 61) is provided in at least two of the plurality of channels (15a, 15b, 15c) and each of the at least two of the plurality of channels (15a, 15b, 15c) is provided with a respective engagement mechanism (45; 61) which can be operated independently of each other.

13. A medical system according to Claim 1, further comprising a supporting device (70; 102) for supporting the over-tube (1) outside the body cavity, the supporting device (70; 102) being adapted to fix the proximal portion of the over-tube (1) in position.

14. A medical system according to Claim 13, wherein the supporting device (70; 102) comprises a first holder (71a, 71b; 104) for supporting the tool (2, 3) at a first portion thereof and a second holder (72a, 72b; 105) for supporting the tool (2, 3) at a second portion thereof.

15. A medical system according to claim 14, wherein the first portion is a midsection portion of the tool (2, 3) and the second portion is a proximal portion (5a, 5b) of the tool (2, 3).

16. A medical system according to Claim 14, wherein the supporting device (70) further comprises a first supporting position adjusting mechanism (76, 77) being capable of changing the supporting position of the over-tube (1) by the first holder (71a, 71b).

17. A medical system according to Claim 14, wherein the supporting device (70) further comprises a second supporting position adjusting mechanism (81, 82) being capable of changing the supporting position of the over-tube (1) by the second holder (72a, 72b).

18. A medical system according to Claim 14, wherein the supporting device (70; 102) can change the relative position of the first holder (71a, 71b; 104) and the second holder (72a, 72b; 105).

19. A medical system according to Claim 18, further comprising an applying mechanism (113; 124) for applying a fixing force for determining and fixing the position of the second holder (105; 122) with respect to the first holder (104; 121) and a releasing mechanism for releasing the fixing force.

20. A medical system according to Claim 19, further comprising an operating mechanism for selectively operating the applying mechanism and the releasing mechanism.

21. A medical system according to Claim 1, wherein the medical system comprises an endoscope (3) as the tool, and wherein the restraining mechanism (45, 54; 61, 62) is a connecting member for connecting the distal portion of the over-tube (1) and the distal portion of the endoscope (3).

22. A medical system according to Claim 1, wherein the tool (2, 3) to be inserted through the over-tube (1) includes an endoscope (3).

23. A medical system according to Claim 1, wherein the tool (2, 3) to be inserted through the over-tube includes an instrument insertion guide tube (2).

## Patentansprüche

1. Ein medizinisches System mit einem Hüllrohr (1), das wenigstens einen Kanal (15a, 15b, 15c) hat, der sich von einem proximalen Abschnitt (5) zu einem distalen Abschnitt (4) erstreckt und eine distale Öffnung (41) aufweist, durch welche ein Werkzeug (2, 3) einführbar und in eine Körperhöhle führbar ist, **dadurch gekennzeichnet, dass**
das Hüllrohr (1) einen Rückhaltemechanismus (45, 54; 61, 62) aufweist, um eine Relativbewegung zwischen dem Werkzeug (2, 3), das durch das Hüllrohr (1) eingeführt ist und dem Hüllrohr (1) einzuschränken, wobei eine gewünschte Position des Werkzeugs (2, 3), das von der distalen Öffnung (41) des Hüllrohrs (1) vorsteht, relativ zum Hüllrohr (1) von einer Bedienungsperson festlegbar ist.

2. Ein medizinisches System nach Anspruch 1, wobei der Rückhaltemechanismus aufweist:
einen Eingriffsmechanismus (45; 61), der an einem distalen Abschnitt des Hüllrohrs angeordnet ist, um das durch das Hüllrohr eingeführte Werkzeug lösbar in Eingriff mit dem Hüllrohr zu bringen; und
einen Betätigungsmechanismus (54-58; 62-65) zur Betätigung des Eingriffs und des Lösens des Eingriffsmechanismus.

3. Ein medizinisches System nach Anspruch 2, wobei der Betätigungsmechanismus (54-58; 62-65) an einer proximalen Seite des Hüllrohrs angeordnet ist.

4. Ein medizinisches System nach Anspruch 2, wobei der Eingriffsmechanismus (45, 54) aufweist:
einen federnden Streifen (46), der am distalen Abschnitt des Hüllrohrs angeordnet ist; und
ein Druckteil (48), um den federnden Streifen (46) gegen das Werkzeug (2, 3) zu drücken, das durch das Hüllrohr (1) eingeführt ist.

5. Ein medizinisches System nach Anspruch 4, wobei der federnde Streifen (46) mit dem Werkzeug (2, 3) in Eingriff gelangt, indem er durch eine Niederdrückung durch das Druckteil (48) verformt wird und den Eingriff beim Aufheben der Niederdrückung durch Rückstellung löst.

6. Ein medizinisches System nach Anspruch 5, wobei das Druckteil (48) den federnden Streifen (46) niederdrückt und das Niederdrücken des federnden Streifens durch eine Gleitbewegung entlang der Innenwand des Kanals (15a, 15b, 15c) des Hüllrohrs (1) aufhebt und wobei der Betätigungsmechanismus (54-58) die Gleitbewegung des Druckteils (48) antreibt.

7. Ein medizinisches System nach Anspruch 2, wobei der Eingriffsmechanismus einen Ballon (61) enthält, der am distalen Ende des Hüllrohrs angeordnet ist.

8. Ein medizinisches System nach Anspruch 7, wobei der Betätigungsmechanismus (62-65) dem Ballon (61) ein Fluid zuführt und dieses hiervon abführt.

9. Ein medizinisches System nach Anspruch 2, wobei die Betätigung des Betätigungsmechanismus (54-58) dem Eingriffsmechanismus über einen Draht (54) übertragen wird.

10. Ein medizinisches System nach Anspruch 2, wobei die Betätigung des Betätigungsmechanismus (62-65) dem Eingriffsmechanismus über eine Röhre (62) übertragen wird, durch welche ein Fluid zuführbar und abführbar ist.

11. Ein medizinisches System nach Anspruch 2, wobei das Hüllrohr (1) eine Mehrzahl von Kanälen (15a, 15b, 15c) enthält, durch welche eine Mehrzahl von Werkzeugen (2, 3) einführbar ist und wobei der Eingriffsmechanismus (45; 61) in wenigstens einem der Kanäle (15a, 15c) angeordnet ist.

12. Ein medizinisches System nach Anspruch 11, wobei der Eingriffsmechanismus (45; 61) in wenigstens zwei der Mehrzahl von Kanälen (15a, 15b, 15c) angeordnet ist und jeder der wenigstens zwei aus der Mehrzahl von Kanälen (15a, 15b, 15c) mit einem entsprechenden Eingriffsmechanismus (45; 61) versehen ist, welche unabhängig voneinander betätigbar sind.

13. Ein medizinisches System nach Anspruch 1, weiterhin mit einer Lagervorrichtung (70, 102) zum Lagern des Hüllrohrs (1) außerhalb der Körperhöhle, wobei die Lagervorrichtung (70, 102) den proximalen Abschnitt des Hüllrohrs (1) an Ort und Stelle festzulegen vermag.

14. Ein medizinisches System nach Anspruch 13, wobei die Lagervorrichtung (70; 102) einen ersten Halter (71a, 71b; 104) zum Lagern des Werkzeugs (2, 3) an einem ersten Abschnitt hiervon und einen zweiten Halter (72a, 72b; 105) aufweist, um das Werkzeug (2, 3) an einem zweiten Abschnitt hiervon zu lagern.

15. Ein medizinisches System nach Anspruch 14, wobei der erste Abschnitt ein mittlerer Abschnitt des Werkzeugs (2, 3) ist und der zweite Abschnitt ein proximaler Abschnitt (5a, 5b) des Werkzeugs (2, 3) ist.

16. Ein medizinisches System nach Anspruch 14, wobei die Lagervorrichtung (70) weiterhin einen ersten Lagerpositionseinstellmechanismus (76, 77) aufweist, der die Lagerposition des Hüllrohrs (1) durch den ersten Halter (71a, 71b) zu ändern vermag.

17. Ein medizinisches System nach Anspruch 14, wobei die Lagervorrichtung (70) weiterhin einen zweiten Lagerpositionseinstellmechanismus (81, 82) aufweist, der die Lagerposition des Hüllrohrs (1) durch den zweiten Halter (72a, 72b) zu ändern vermag.

18. Ein medizinisches System nach Anspruch 14, wobei die Lagervorrichtung (70; 102) die Relativposition des ersten Halters (71a, 71b; 104) und des zweiten Halters (72a, 72b; 105) zu ändern vermag.

19. Ein medizinisches System nach Anspruch 18, weiterhin mit einem Anlegemechanismus (113; 124), um eine Haltekraft zum Bestimmen und Festlegen der Position des zweiten Halters (105; 122) bezüglich des ersten Halters (104; 121) anzulegen, sowie mit einem Freigabemechanismus zum Aufheben der Haltekraft.

20. Ein medizinisches System nach Anspruch 16, weiterhin mit einem Betätigungsmechanismus zum selektiven Betätigen des Anlegemechanismus und des Freigabemechanismus.

21. Ein medizinisches System nach Anspruch 1, wobei das medizinische System ein Endoskop (3) als das Werkzeug aufweist und wobei der Rückhaltemechanismus (45, 54; 61, 62) ein Verbindungsbauteil zur Verbindung des distalen Abschnitts des Hüllrohrs (1) und des distalen Abschnitts des Endoskops (3) ist.

22. Ein medizinisches System nach Anspruch 1, wobei das über das Hüllrohr (1) einzuführende Werkzeug (2, 3) ein Endoskop (3) beinhaltet.

23. Ein medizinisches System nach Anspruch 1, wobei das über das Hüllrohr (1) einzuführende Werkzeug (2, 3) eine Instrumenteneinführ-Führungsröhre (2) enthält.

## Revendications

1. Système médical comprenant un surtube (1) ayant au moins un canal (15a, 15b, 15c) s'étendant d'une partie proximale (5) jusqu'à une partie distale (4) et ayant une ouverture distale (41) à travers laquelle un outil (2, 3) est apte à être inséré et guidé à l'intérieur d'une cavité corporelle,
**caractérisé en ce que** le surtube (1) comprend un mécanisme de restriction (45, 54 ; 61, 62) pour restreindre un mouvement relatif entre l'outil (2, 3) inséré à travers le surtube (1) et le surtube (1), d'où il résulte qu'une position désirée de l'outil (2, 3) se projetant à partir de l'ouverture distale (41) du surtube (1) est fixable par rapport au surtube (1) par un opérateur.

2. Système médical selon la revendication 1, dans lequel le mécanisme de restriction comprend :
un mécanisme d'engagement (45 ; 61) prévu au niveau d'une partie distale du surtube pour engager de façon libérable l'outil inséré à travers le surtube avec le surtube ; et
un mécanisme opérationnel (54-58 ; 62-65) pour un engagement opérationnel et une libération du mécanisme d'engagement.

3. Système médical selon la revendication 2, dans lequel le mécanisme opérationnel (54-58 ; 62-65) est prévu sur un côté proximal du surtube.

4. Système médical selon la revendication 2, dans lequel le mécanisme d'engagement (45, 54) comprend :
une bande résiliente (46) disposée au niveau de la partie distale du surtube ; et
un élément de pression (48) pour presser la bande résiliente (46) contre l'outil (2, 3) inséré à travers le surtube (1).

5. Système médical selon la revendication 4, dans lequel la bande résiliente (46) engage l'outil (2, 3) en étant déformée par la dépression par l'élément de pression (48), et libère l'engagement en étant restaurée par la libération de la dépression.

6. Système médical selon la revendication 5, dans lequel l'élément de pression (48) presse la bande résiliente (46) et libère la dépression de la bande résiliente par un mouvement de glissement le long de la paroi intérieure du canal (15a, 15b, 15c) du surtube (1), et dans lequel le mécanisme opérationnel (54-58) opère le mouvement de glissement de l'élément de pression (48).

7. Système médical selon la revendication 2, dans lequel le mécanisme d'engagement inclut un ballonnet (61) disposé au niveau de l'extrémité distale du surtube.

8. Système médical selon la revendication 7, dans lequel le mécanisme opérationnel (62-65) amène et décharge un fluide dans et du ballonnet (61).

9. Système médical selon la revendication 2, dans lequel l'opération du mécanisme opérationnel (54-58) est transmise au mécanisme d'engagement par l'intermédiaire d'un fil (54).

10. Système médical selon la revendication 2, dans lequel l'opération du mécanisme opérationnel (62-65) est transmise au mécanisme d'engagement par l'intermédiaire d'un tube (62) à travers lequel un fluide est amenable et déchargeable.

11. Système médical selon la revendication 2, dans lequel le surtube (1) inclut une pluralité de canaux (15a, 15b, 15c) à travers lesquels une pluralité d'outils (2, 3) peuvent être insérés, et dans lequel le mécanisme d'engagement (45 ; 61) est prévu dans au moins l'un des canaux (15a, 15b, 15c).

12. Système médical selon la revendication 11, dans lequel le mécanisme d'engagement (45 ; 61) est prévu dans au moins deux de la pluralité de canaux (15a, 15b, 15c) et chacun des au moins deux de la pluralité de canaux (15a, 15b, 15c) est prévu avec un mécanisme d'engagement (45 ; 61) respectif qui peut être opéré indépendamment l'un de l'autre.

13. Système médical selon la revendication 1, comprenant en outre un dispositif de support (70 ; 102) pour supporter le surtube (1) à l'extérieur de la cavité corporelle, le dispositif de support (70 ; 102) étant adapté pour fixer la partie proximale du surtube (1) en position.

14. Système médical selon la revendication 13, dans lequel le dispositif de support (70 ; 102) comprend un premier support (71a, 71b ; 104) pour supporter l'outil (2, 3) au niveau d'une première partie de celui-ci et un deuxième support (72a, 72b ; 105) pour supporter l'outil (2, 3) au niveau d'une deuxième partie de celui-ci.

15. Système médical selon la revendication 14, dans lequel la première partie est une partie médiane de l'outil (2, 3) et la deuxième partie est une partie proximale (5a, 5b) de l'outil (2, 3).

16. Système médical selon la revendication 14, dans lequel le dispositif de support (70) comprend en outre un premier mécanisme d'ajustement de position de support (76, 77) apte à changer la position de support du surtube (1) par le premier support (71a, 71b).

17. Système médical selon la revendication 14, dans lequel le dispositif de support (70) comprend en outre un deuxième mécanisme d'ajustement de position de support (81, 82) apte à changer la position de support du surtube (1) par le deuxième support (72a, 72b).

18. Système médical selon la revendication 14, dans lequel le dispositif de support (70 ; 102) peut changer la position relative du premier support (71a, 71b ; 104) et du deuxième support (72a, 72b ; 105).

19. Système médical selon la revendication 18, comprenant en outre un mécanisme d'application (113 ; 124) pour appliquer une force de fixation pour déterminer et fixer la position du deuxième support (105 ; 122) par rapport au premier support (104 ; 121) et un mécanisme de libération pour libérer la force de fixation.

20. Système médical selon la revendication 19, comprenant en outre un mécanisme opérationnel pour opérer sélectivement le mécanisme d'application et le mécanisme de libération.

21. Système médical selon la revendication 1, dans lequel le système médical comprend un endoscope (3) en tant que l'outil, et dans lequel le mécanisme de restriction (45, 54 ; 61, 62) est un élément de connexion pour connecter la partie distale du surtube (1) et la partie distale de l'endoscope (3).

22. Système médical selon la revendication 1, dans lequel l'outil (2, 3) destiné à être inséré à travers le surtube (1) inclut un endoscope (3).

23. Système médical selon la revendication 1, dans lequel l'outil (2, 3) destiné à être inséré à travers le surtube inclut un tube de guidage d'insertion d'instrument (2).
